Europäisches Patentamt

European Patent Office

Office européen des brevets

(19)

(11) Numéro de publication: **0 352 147**
**A2**

(12)

# DEMANDE DE BREVET EUROPEEN

(21) Numéro de dépôt: **89401204.6**

(22) Date de dépôt: **27.04.89**

(51) Int. Cl.5: **A 61 K 31/35**
**A 61 K 31/70**

(30) Priorité: **23.06.88 FR 8808434**

(43) Date de publication de la demande:
**24.01.90 Bulletin 90/04**

(84) Etats contractants désignés:
**AT BE CH DE ES GB GR IT LI LU NL SE**

(71) Demandeur: **FONDATION POUR L'ENCOURAGEMENT A LA RECHERCHE MEDICALE**
**C% Arcomm Treuhand Anstalt B.P. 745**
**Vaduz (LI)**

(72) Inventeur: **Beljanski, Mirko**
**46, boulevard de Port Royal**
**F-75005 Paris (FR)**

(74) Mandataire: **Pinguet, André**
**Cabinet de Proprieté Industrielle CAPRI 28 bis, avenue Mozart**
**F-75016 Paris (FR)**

(54) **Utilisation de la Naringine, de la Naringénine ou leurs dérivés pour faire une composition pharmaceutique anticancéreuse.**

(57) Naringine et Naringenine ont été découvertes être des agents pharmaceutiques inhibant avec sélectivité la croissance des cellules cancéreuses en présence de cellules saines. Ces substances sont aussi efficaces contre des cellules cancéreuses résistantes à la chimiothérapie et à la radiothérapie. Il a été montré que Naringine et Naringenine contractent sélectivement les chaines d'ADN des cellules tumorales et non les ADN des cellules saines . Les composés dont il est ici question présentent une activité antivirale. Une méthode nouvelle de recouvrement de la Naringine/Naringenine à partir de sources naturelles végétales est décrite.

EP 0 352 147 A2

Description

## COMPOSITION PHARMACEUTIQUE ANTICANCEREUSE ET METHODE D'UTILISATION DE L'INVENTION.

Le nombre des cancers solides , du sang ou du système lymphatique stagne et même augmente cependant que les traitements par la chimiothérapie donnent des résultats très insuffisants . La difficulté majeure vient du fait que les agents chimiothérapeutiques sont toxiques et mêmes parfois léthaux . Ils inhibent la prolifération non seulement des cellules malignes mais également celle des cellules normales , ce qui rend nécessaire la découverte d'agents sélectifs capables d'agir par eux mêmes , ou en combinaison afin que l'effet inhibiteur soit prédominant vis à vis des cellules indésirables . Disposer d'agents hautement sélectifs tout en ayant le minimum d 'effet secondaire est un objectif important de la recherche sur le cancer.

Le même problème d'absence de sélectivité et d'effets secondaires indésirables se manifeste aussi lors de l'utilisation de la radiothérapie .

Des efforts considérables sont également déployés afin de trouver des entités chimiques qui seraient actives contre les virus , cause de maladies des hommes, animaux et plantes. Les antitées antivirales connues sont également non sélectives et en nombre bien inférieur à celles connues pour agir contre les bactéries et les champignons.

Dans les cas de malignité ou des virus , il est souhaitable de trouver des entités actives et sélectives agissant au niveau des cellules anormales , c'est à dire affectant l'ADN ou les enzymes impliqués dans la prolifération de la cellule maligne ou virosée .

### Brève description de l'invention

J'ai trouvé que la Naringine , Naringenine ou leurs dérivés acceptables sur le plan pharmaceutique , inhibent la croissance des cellules cancéreuses .J'ai aussi trouvé que ces composés ne portent pas de préjudice conséquent aux cellules normales . Cette sélectivité entre les cellules cancéreuses et normales font de Naringine et Naringenine des agents thérapeutiques efficaces pour le traitement des cancers .

Pour être concret , Na ringine, Naringenine ou leurs esters , sels et dérivés sous une forme pharmaceutique acceptable , ou un mélange de l'un ou de plusieurs d'entre eux sont amalgamés avec un support pharmaceutique acceptable . La composition pharmaceutique résultante est alors séparée en unité de dosage et une ou plusieurs unités sont administrées aux patients à un temps donné . Le dosage , le mode d'administration ainsi que sa fréquence et durée , varient selon le patient , et sont choisis par le médecin afin d'assurer le meilleur contact entre les cellules cancéreuses et le produit agissant en concentration efficace .

Un certain nombre de publications mentionnent l'emploi de Naringine dans des applications thérapeutiques. Très peu mentionnent l'usage de la Naringine en carcinotherapie. Le brevet japonais A-62 273 915 cité dans les chemical abstracts vol. 109, n° 1 du 4 juillet 1988 page 812, décrit une application dans laquelle le produit n'agit pas directement sur les cellules cancéreuses. La Naringine est utilisée pour induire une substance destinée à agir sur la tumeur. On a toutefois démontré depuis que cette substance stimule la multiplication des virus, notamment du Sida.

Un article "Antiviral effect of flavonoids on Human Viruses" paru dans "Journal of Medical Virology, 15:71-79 (1985)" explique que la Naringenine n'a pas d'effet sur l'infectivité ou la réplication de certains virus étudiés. Ceci, non seulement ne peut conduire à la présente invention mais dissuade le chercheur de s'engager dans cette voie.

Un article "Partial transition-state inhibitors of glyoxalase 1 from human erythrocytes, yeast and rat liver" paru dans "Biochemica and Biophysica acta" vol. 829, 1985, pages 109 - 118, décrit l'action de plusieurs substances sur la glyoxalase des levures, dont la Naringine, et indique que cela pourrait conduire à des inhibiteurs de cellules cancéreuses. Mais il n'y a pas de démonstration, et surtout pas de sélectivité entre les cellules normales et les cellules tumorales.

Une publication "Antivirale Wirkung von Pflanzeninhalts stoffen" du Departement Biochimie thérapeutique, du centre de chimie biologique de l'Université de Francfort/Main (1978) mentionne l'action de divers flavonoides, dont la naringine contre les virus en culture et indique un effet de 8 à 18 % pendant 24 heures, ce qui est totalement insuffisant et inapplicable.

Un autre aspect de l'invention porte sur le traitement d'un patient dont les cellules cancéreuses sont résistantes à la chimiothérapie ou à la radiothérapie par un des agents de l'invention . J'ai trouvé que ces agents peuvent aussi être efficaces contre les cellules cancéreuses qui sont résistantes à la chimiothérapie et/ou à la radiothérapie.

Dans un autre aspect de l'invention , il a été trouvé que la Naringine et la Naringenine , ou leurs dérivés , présentent un effet synergique lorsqu 'ils sont administrés aux animaux ou aux patients subissant la chimiothérapie conventionnelle.

Un autre aspect de l'invention réside dans la mise en évidence que la Naringine et la Naringenine provoquent la contraction , c'est à dire la fermeture des chaines d'ADN des cellules cancéreuses mais non celle des chaines d'ADN des cellules normales . Les agents de cette invention agissent sur les sites d'initiation de l'ADN des cellules cancéreuses et arrètent également leur élongation. On peut concevoir que cela cause en totalité ou en partie l'inhibition de la croissance des cellules cancéreuses . La fermeture des chaines d'ADN prévient ou inhibe l'addition d'unités de désoxyribonucleotides à la molécule d'ADN.

Le fait que la Naringine et la Naringenine contractent les chaines d'ADN des cellules cancéreuses suggère que dans le Syndrome de l'Immuno dépression (SIDA) provoqué par le virus HIV , la Naringine et la Naringenine peuvent prévenir la formation de particules virales à partir des "proto-oncogènes" qui doivent être modifiés pendant l'infection en vue de la production de virus . Ainsi , Naringine et Naringenine peuvent être utiles dans le traitement du SIDA.

En ce qui concerne les plantes et les animaux subissant l'infection virale , Naringine et Naringenine administrées à l'hôte inhibent la prolifération virale . Ceci peut être du à leur activité inhibitrice de certaines enzymes présentes ou produites par le virus .

Ainsi une autre considération de l'invention comporte une méthode pour l'extraction de la Naringine et de la Naringenine à partir de sources végétales naturelles.

## Les illustrations

Figure 1 représente le spectre d'absorption en lumière ultraviolette de la Naringine commerciale et celui de la Naringine préparée selon la méthode exposée dans la présente invention .

Figure 2 montre les effets de la Naringine lorsqu'elle est testée sur des cellules en culture in vitro , cellules humaines cancéreuses et normales

Figure 3 montre l'effet de la Naringine sur la synthèse in vitro de l'ADN provenant des cellules normales et cancéreuses .

Figure 4 montre l'effet de la Naringine sur la synthèse in vitro de l'ADN cancéreux lorsqu'elle est ajoutée au temps zero de la réaction et lorsqu'elle est ajoutée après que la réaction de synthèse ait démarrée .

Figure 5 montre l'effet de la Naringine et de la Naringenine dans la contraction des chaines d'ADN des cellules cancéreuses alors qu'elles sont sans effet sur la contraction des ADN des cellules normales , le degré de contraction étant démontré par l'hypochromicité.

Figure 6 montre l'effet de la Naringine et de la Naringenine sur l'activité de la Terminale désoxynucleotidyl transférase (TdT ).

Figure 7 montre une feuille de Tabac infectée par le virus de la mosaïque du Tabac (VMT), un côté de la feuille ayant été traité et l'autre pas.

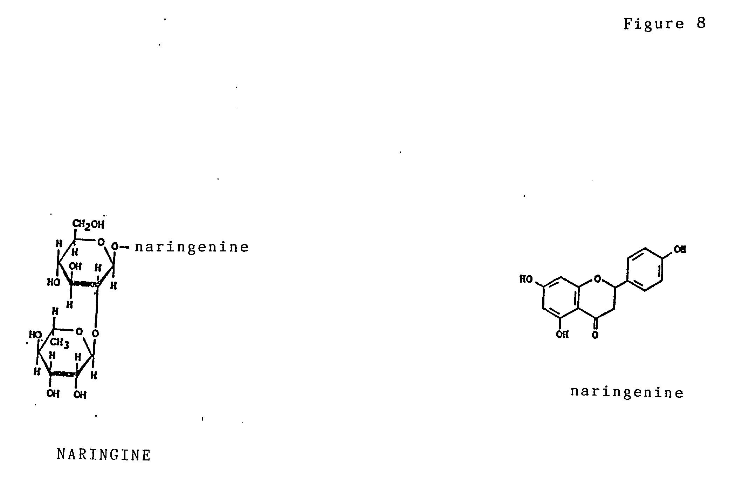

Figure 8 montre la structure chimique de la Naringine et celle de la Naringenine, toutes deux étant connues dans la littérature chimique.

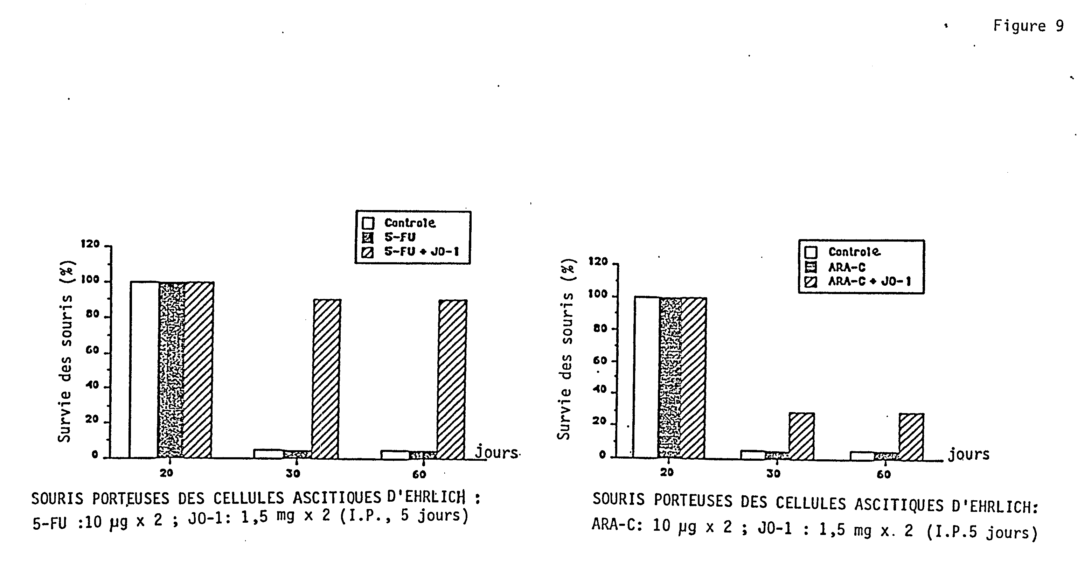

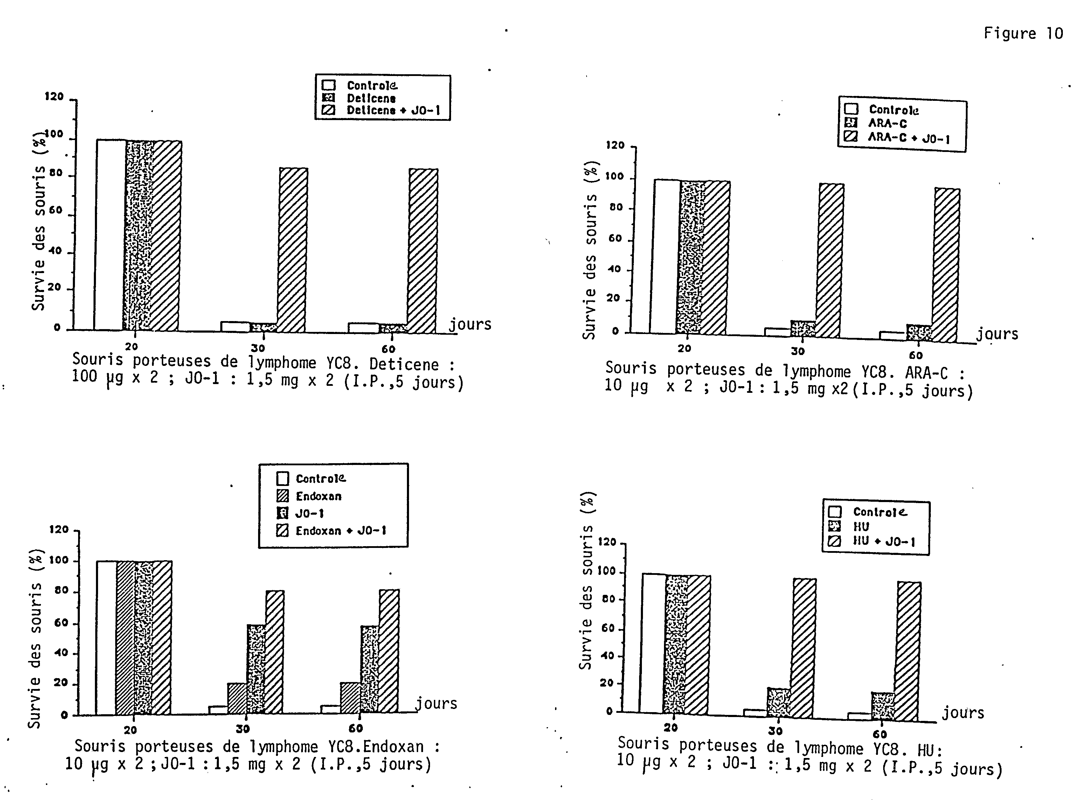

Figure 9 et 10 montrent la synergie d'action entre des antimitotiques classiques et la Naringine isolée selon la méthode exposée dans la présente invention (Exemple 1) . 5-FU = 5-fluorouracile ; JO-1 = Naringine exemple 1 ; ARA-C = arabinoside cytosine ; Endoxan = cyclophosphamide ; HU = hydroxy-urée .

Tableau 1 illustre la survie des souris porteuses de cellules cancéreuses (lymphome YC8 ), non traitées et traitées par la Naringine commerciale ( voie i.p. ) et Naringine exemple 1 .

Tableau 2 illustre la survie des souris porteuses de cellules cancéreuses (lymphome YC8) non traitées et traitées par la Naringine Exemple 1 ( voie i.m.).

Tableau 3 illustre la survie des souris porteuses de cellules cancéreuses (ascite d'Ehrlich), non traitées et traitées par la Naringine selon l'Exemple 1 (voie i.p. )

Tableau 4 illustre la survie des souris porteuses de cellules cancéreuses ( lymphome YC8) , non traitées et traitées par la Naringenine commerciale ( voie i.p.) .

## Description détaillée de l' Invention

Comme leurs dérivés , la Naringine et la Naringenine semblent agir de deux façons différentes sur l'ADN des cellules cancéreuses . Premièrement , ces substances contractent , voire ferment les chaines de ce type d'ADN au niveau des points d'initiation , lors de la synthèse de la chaine . Deuxièmement , elles contractent , ou ferment , les chaines d' ADN en voie de synthèse (élongation ). Ces effets sont probablement dus au fait que les chaines complémentaires de l'ADN fixent la Naringine ou la Naringenine en liaisos hydrogènes (ou covalentes ) . La fermeture des chaines d'ADN des cellules normales ne se produit pas . On peut penser que ces phénomènes sont la cause essentielle de l'inhibition sélective de la croissance des cellules cancéreuses , phénomènes qui ne se produisent pas lorsque des cellules normales sont exposées à l'action de la naringine et de la naringenine. Cependant , je ne souhaite pas être lié par cette théorie , ni par aucune autre théorie .

Une quantité de Naringine, de Naringenine, ou d'ester ou de sels de ces substances , en cela active pour inhiber la croissance , c'est à dire la prolifération des cellules cancéreuses , est administrée à l'animal infecté par des cellules cancéreuses . Une solution de ces produits peut être administrée par voie intraveineuse , intrapéritonéale , intramusculaire ou par voie orale sous forme de comprimés ou de gélules , ainsi que par voie rectale , mais en laissant au médecin traitant le soin de choisir le mode d'administration le plus souhaitable selon les circonstances.

Pour les humains , les dosages seront généralement dans les limites suivantes , mais le médecin peut, selon des cas particuliers , augmenter ou diminuer ces doses .

Per os , c'est à dire par voie orale , 1 à 3 grammes de produit/jour . L'extrait acétonique de naringine peut être mélangé avec la poudre de cellulose , séché à 60 - 80 °C pendant plusieurs heures , puis pulvérisé dans un " mixer " et placé dans les gélules , chacune contenant environ 0.5 gramme de Naringine. Deux à six gélules peuvent être prises par jour sans aucun inconvénient durant plusieurs mois . Les dosages seront à peu près les mêmes pour la voie rectale .

La Naringine peut être administrée par voie intramusculaire sans inconvénient aussi bien chez les animaux que chez les humains. Pour ces derniers , une dose intramusculaire et journalière de 500 - 1000 milligrammes pendant plusieurs jours consécutifs peut être utilisée . La Naringine peut être aussi utilisée en solution contenant de faibles quantités d'alcool éthylique ou autre solvent , tel le dimethylsulfoxide (DMSO).

Pour l'injection ou perfusion intraveneuses , 250-1000 milligrammes seront généralement bien tolérés . Le produit peut être dissous dans une solution fraiche de DMSO puis dilué avec une solution aqueuse physiologique jusqu'à une concentration finale de DMSO de 5 à 10 % .

Des dosages du même ordre que ceux utilisés pour lutter contre les cellules cancéreuses seront utilisées pour lutter contre les affections virales ; les mêmes modes d'administration pourront être employés.

L'utilisation de filtres millipores comme décrit dans l'Exemple 1 est pratiqué avant toute injection intraveineuse ou perfusion. Pour l'utilisation orale ou rectale , il suffit d'utiliser l'extrait soluble (phase acétone et/ou ether , après qu'ait été ajusté le pH à des valeurs neutres . En effet , il n'y a pas de toxicité du sirop commercial utilisé comme source de substance , l'extrait de Naringine(phase acétone et/ou ether) ne présente pas d'effet toxique chez les animaux . L'extrait de Naringine concentré peut être mélangé avec la poudre de cellulose, séché puis pulvérisé et mis directement en gélules de manière à ce que chaque gélule contienne une unité de dosage appropriée ( voie orale ) , ou amalgamé à des cires prévues à cet usage pour la forme suppositoire (voie rectale ).

En prévention d'infections virales des plantes ou en traitement post-infection , le produit actif pourrait être appliqué avec un transporteur physiologique acceptable , liquide ou solide, particulièrement adapté afin de favoriser l'adhésion sur les feuilles ou autres parties de la plante ( 50 à 200 microgrammes par feuille ).

## Extraction de Naringine des plantes

### Exemple 1 Hippophae Rhamnoides

Un sirop commercial contenant de fortes quantités d'extrait de baies de la plante Hippophae Rhamnoides a été utilisé comme matériel de départ .

A 500 millilitres de sirop , on ajoute de l'acide chlorhydrique (concentration finale 1 N ) et le mélange est chauffé dans un bécher pendant quinze minutes à 100°C. Après refroidissement , le matériel hydrolysé est additionné d'un excès d'acétone (3 à 4 fois le volume ) . Le mélange est agité à la température du laboratoire (agitation mécanique) . Après quelques instants de repos , deux phases apparaissent . La phase supérieure (acétone ) est devenue d'un brun-jaune soutenu . Elle est prélevée et gardée. Le traitement de la phase aqueuse par de l'acétone est répété plusieurs fois jusqu'à ce que l'acétone devienne très pâle . Ensuite , plusieurs extractions sont pratiquées de la même manière avec de l'éther en vue d'extraire le maximum d'agent actif (Naringine et naringenine ).

Les phases acétones sont réunies entre elles , et distillées. Les phases éther sont réunies entre elles et distillées , les solvents pouvant servir à d'ultérieures extractions .

Les résidus acétone et éther sont réunis , le pH ajusté au voisinage de 7,0 NaOH ou KOH ) .

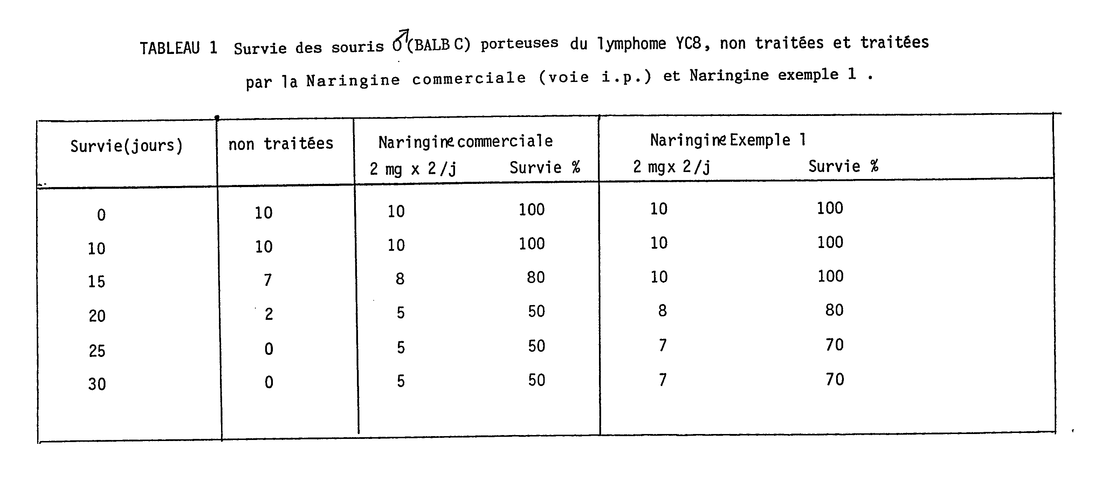

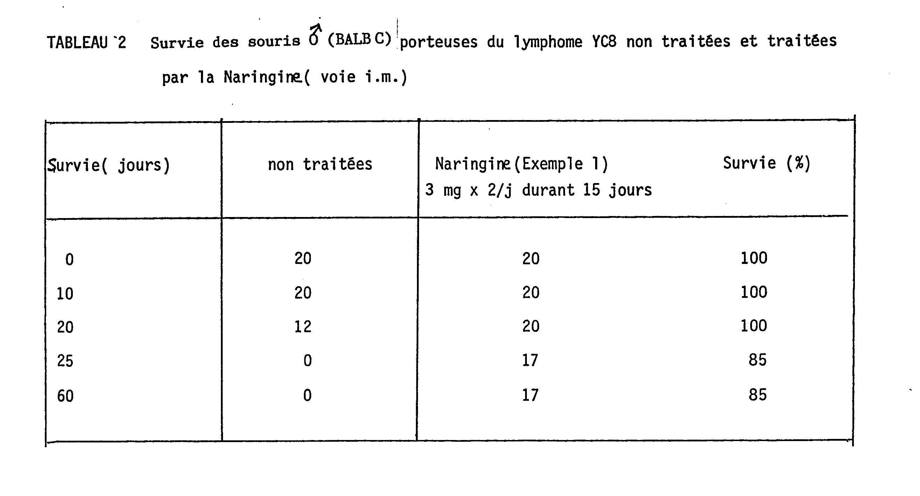

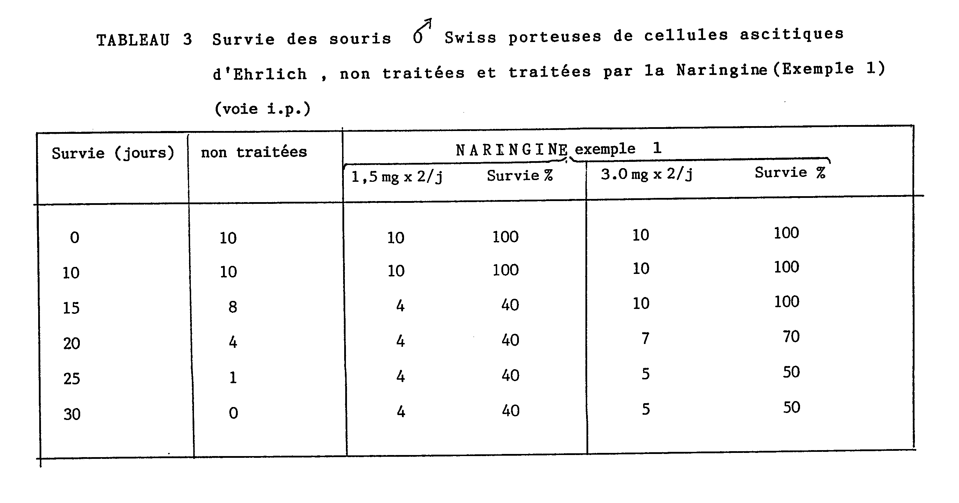

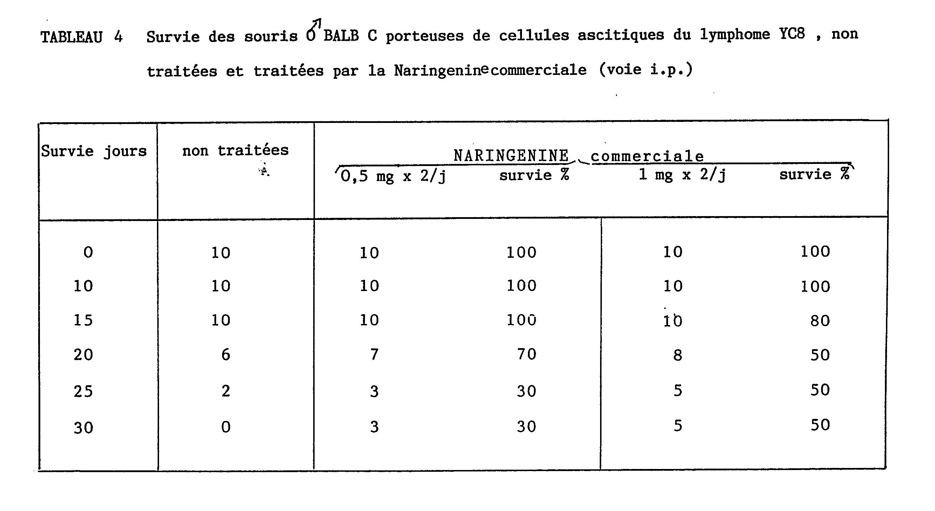

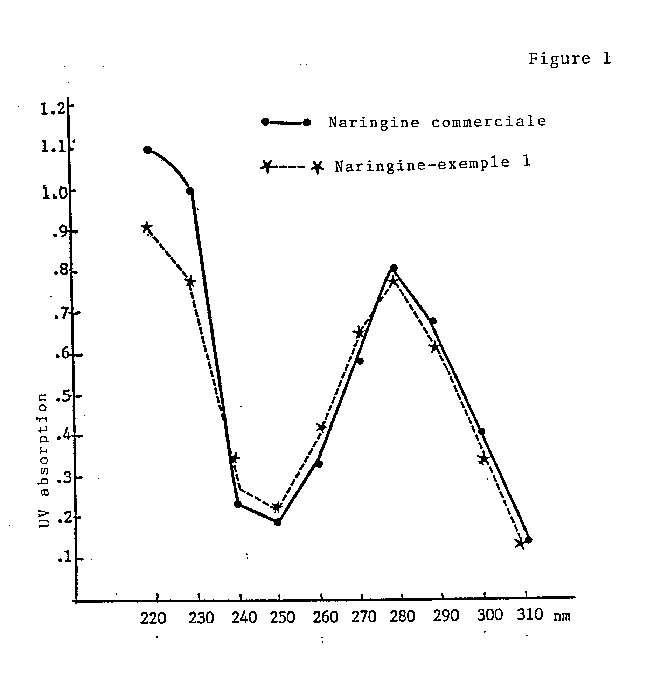

Selon la forme qui sera donnée au produit et le mode d'administration , celui-ci sera ou non ultérieurement purifié. Si l'on décide de le purifier davantage , les résidus acétone/ether sont repris par un mélange eau-ethanol (1 : 1 ) , le pH ajusté à 10 - 12 et le matériel filtré sur des filtres millipores de 0.45 μ . Le matériel restant sur millipore contient l'essentiel de la Naringine/Naringenine. Les filtres sont alors incubés avec de l'alcool ethylique à 95° dans un bécher et légèrement chauffés .L'extraction par l'alcool ethylique est répétée plusieurs fois . La quantité de Naringine dissoute dans l'éthanol est déterminée par l'absorption en lumière ultraviolette (UV) à différentes longueurs d'onde (nm) et donne un spectre caractéristique , illustré dans la figure 1 . La préparation contient essentiellement la naringine et la naringenine est en faible quantité . Peu ou pas de vitamine C , de sucre ou de lipides sont présents dans une telle préparation .

La comparaison avec la Naringine commerciale utilisée comme étalon (dissolution dans l'alcool ethylique ) est faite selon plusieurs critères . Le spectre ultraviolet d'absorption de 220 à 310 nm est déterminé en utilisant 4 à 20 μg (microgrammes) de naringine/millilitre. Le spectre du produit isolé selon l'exemple 1 coincide essentiellement avec celui obtenu avec le produit de référence . Illustration dans la figure 1 . La Naringenine a pratiquement le même spectre au maximum d'absorption (290 nm ) que la solution de référence.

Sur papier de chromatographie ou sur plaque de gel de Silice, la naringine isolée selon l'exemple 1 migre comme le fait la naringine commerciale en utilisant l'alcool ethylique comme solvent . Les spots (taches) sont détectés par la lumière UV et sont , dans les deux cas , proches du front de solvent.

Le même procédé d'extraction et de purification a été directement appliqué aux fruits de l'Hippophae .

La préparation selon l'exemple 1 est plus soluble dans l'eau et possède une meilleure activité biologique , comparaison faite avec les composés commerciaux . Cette différence est probablement due à la présence de petites quantités de sucre ou lipides dans la préparation.

La Naringine préparée selon la méthode illustrée dans cet exemple porte le nom de " JO-1 " et les tests ont été réalisés sous ce même nom.

### Exemple 2. Citrons verts

La peau de citrons verts est prélevée , broyée au "mixer " en mélange aqueux. Pour l'isolement de la Naringine , le même procédé que celui cité ci-dessus (exemple 1) a été utilisé. Certains pamplemousses peuvent aussi servir de source d 'extraction de la Naringenine ou de la Naringine .

### Exemple 3. Racines d 'endives (chicorum endivia )

Les racines d'endives poussées en terre sont lavées puis la couche externe noire est enlevée (grattage). Les racines sont lavées , coupées en morceaux, broyées avec de l'eau afin de produire un mélange épais qui est mis à bouillir 30 min (100°C); Le liquide est alors filtré sur tissus , les débris végétaux sont réextraits dans les mêmes conditions à plusieurs reprises. Les filtrats sont réunis , concentrés , puis hydrolysés avec l'acide chlorhydrique (concentration finale 1 N ) pendant 10 min à 100°C , puis refroidis . Les étapes suivantes de purification sont les mêmes que celles décrites dans l'exemple 1 .

Dans tous les exemples cités dans la présente invention , la Naringine provient de baies d'hyppophae Ramnoïdes ( une préparation commerciale ) à partir de laquelle nous avons extrait le produit selon l'exemple 1 .

## Exemple 4

L'effet de la Naringine et/ou de la Naringenine a été détecté selon une méthode in vitro mise au point et permettant de montrer l'action d'une substance donnée dans le processus de réplication de l'ADN provenant soit de tissus normaux soit de tissus cancéreux, comme décrit dans le brevet français d'application n° 7728208 du 19 septembre 1977 , ce qui correspond au brevet américain n° 4,264,729 délivré le 28 avril 1981 .

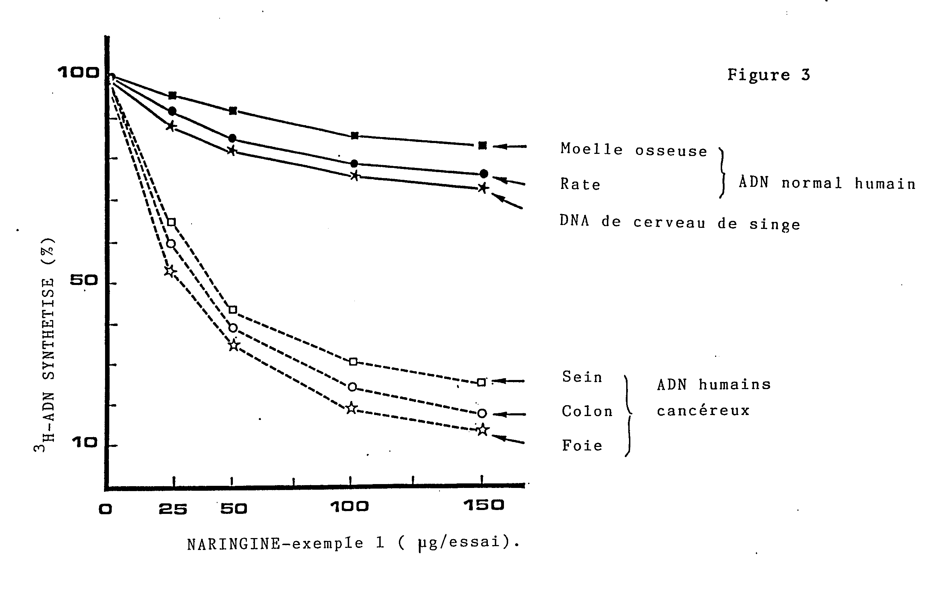

Les résultats concernant la Naringine sont présentés dans la figure 3. Ils montrent que ce produit inhibe in vitro fortement la synthèse des ADN de différents tissus cancéreux humains c'est à dire sein , colon et foie , alors que ce même produit n'a pratiquement pas d'action sur la synthèse de l'ADN des tissus sains : moelle osseuse et rate humaines et cerveau de singe . Cela montre que la Naringine interagit spécifiquement avec l'ADN des tissus cancéreux et non pas avec l'ADN polymérase. Il en est de même avec la Naringenine. Les ADN utilisés ont été isolés à partir de tissus cancéreux et normaux humains , fournis par différents hopitaux comme indiqué dans IRCS Medical Science 14 : 809-810,1986. Le tissu sain du cerveau de singe provient d'un Macaca mulatta.

## Exemple 5

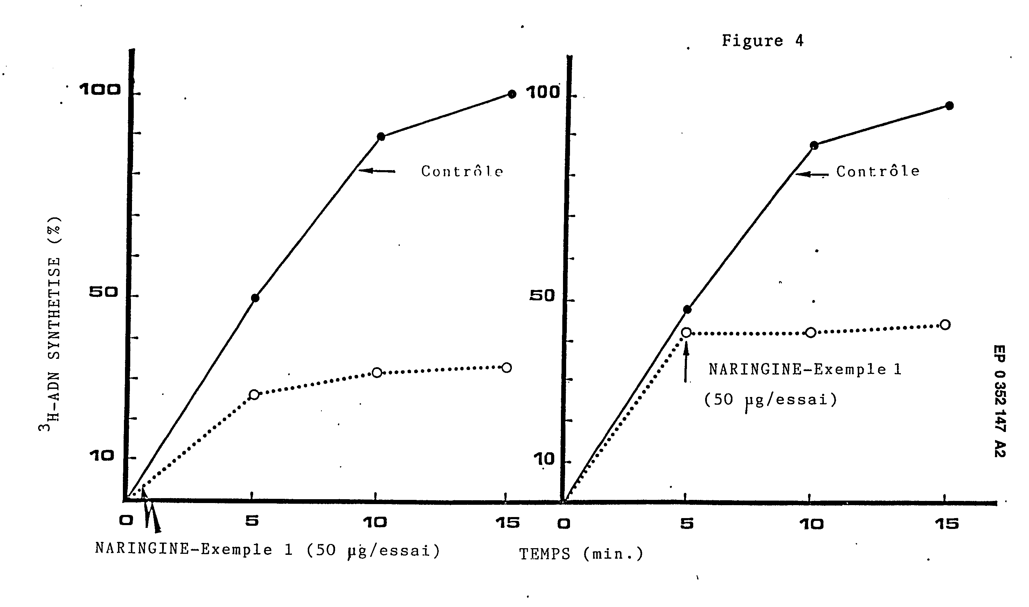

La même méthode est appliquée à la détermination du taux d'ADN marqué au tritium et synthétisé durant un temps défini afin de montrer l'effet d'introduction de Naringine à différents moments de la réaction. Les résultats , présentés dans la figure 4 , montrent que la Naringine agit sur les sites d'initiation de l'ADN lorsqu'elle est introduite au temps zero de l'incubation , et qu'elle arrête aussi l'élongation de la chaine d'ADN si elle est introduite après le début de la réaction .

## Exemple 6

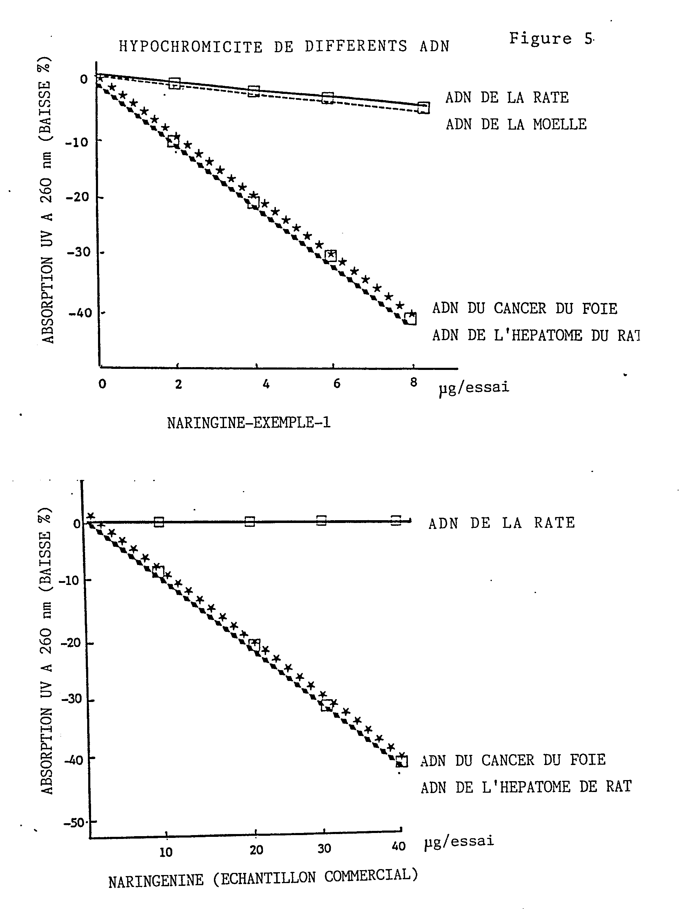

La Naringine est préparée selon la méthode décrite dans l'exemple 1 , et un échantillon commercial de Naringine (préparé par la Maison Roth, France ), est utilisé comparativement. Les résultats , présentés dans la figure 5 , montrent que les deux échantillons de Naringine (et/ou de Naringenine) contractent les chaines d'ADN des cellules cancéreuses mais n'ont pas d'effet - ou n'ont qu'un très faible effet - sur l'ADN des cellules normales . Les résultats sont exprimés en diminution de l'absorption en lumière ultraviolette (hypochromicité) en présence de différentes concentrations de produit. L'hypochromicité correspond à la contraction des chaine d'ADN tandis que l'hyperchromicité correspond à son ouverture, c'est à dire à la rupture des liaisons hydrogènes qui normalement tiennent ensemble les deux brins d'ADN . L'ADN de la rate et celui de la moelle osseuse , utilisés dans ce test provenaient de cellules de tissus sains humains , rate et moelle osseuse .

L'absorption en lumière ultraviolette à la longueur d'onde de 260 nm de différents ADN provenant de cellules saines et cancéreuses s'effectue à la température du laboratoire . 20 µg (microgrammes ) d'ADN (acide désoxyribonucléique)sont dissous dans 1 ml de tampon Tris-HCl ( $10^{-2}$ molaire , pH ajusté à 7.3 , solution faite en utilisant de l'eau distillée stérile ). L'absorption est mesurée avant et après addition soit de Naringine, soit de Naringenine. La cuvette témoin contient un taux équivalent de Naringine ou de Naringenine mais pas d'ADN . Le contact entre l'ADN et la substance dure environ une minute (agitation très douce ) . L'absorption à 260 nm est alors mesurée encore . Les résultats sont exprimés sous forme de diminution de l'absorption U.V. (%)

## Exemple 7

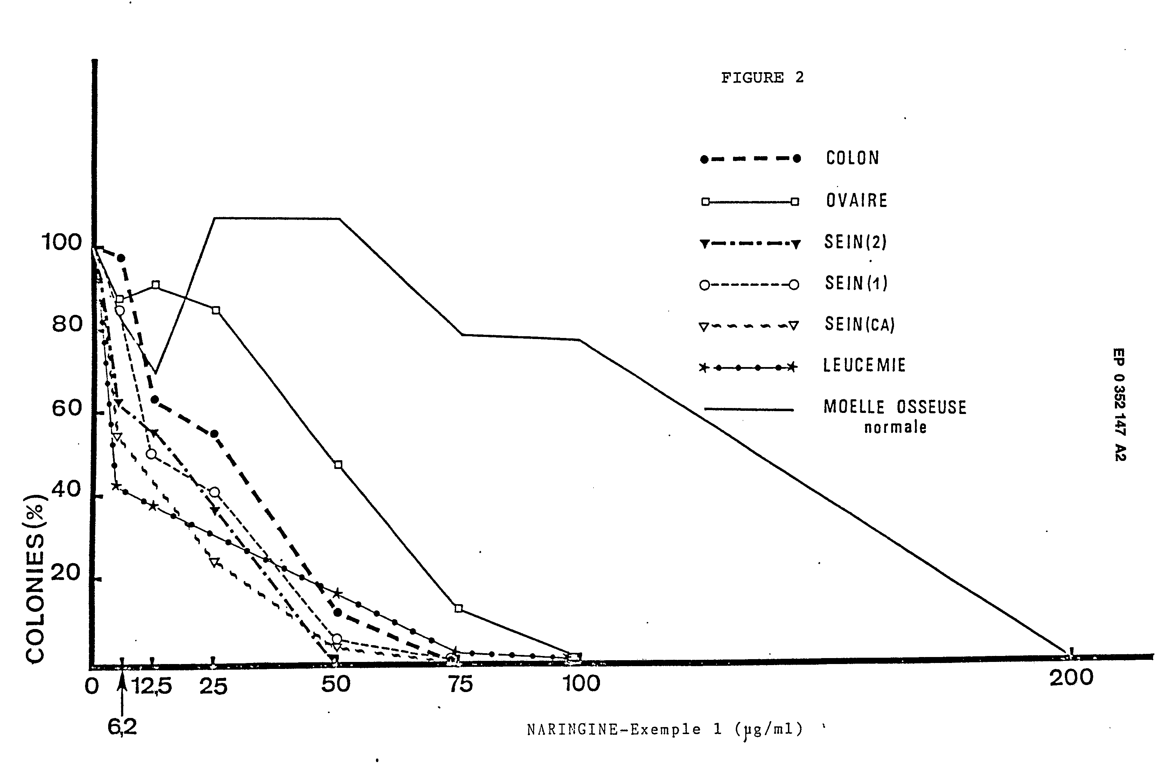

La Naringine préparée selon l' Exemple 1 fut utilisée pour déterminer son effet sur des cellules en culture in vitro , cellules saines et cancéreuses d'origine humaine selon la méthode Shoemaker R.N. et al. Cancer Research 45: 2145-2153,1985).Le milieu de culture contenait 100 µg de penicilline et de streptomycine afin d'éviter toute contami nation. La figure 2 présente les résultats de ce test , montrant l'effet différentiel de différentes concentrations de Naringine. La Naringine inhibe fortement la croissance des différentes cellules cancéreuses humaines et n'a que très peu d'effet sur les cellules saines provenant de la moelle osseuse humaine , et ceci malgré la grande sensibilité que présentent les cellules hématologiques face à l'action des agents chimiotherapeutiques en général .

Il est important de noter que les cellules cancéreuses de l'ovaire ainsi que les cellules leucémiques utilisées dans cet exemple étaient des cellules résistantes à la chimiothérapie ,

Ces résultats pourraient indiquer que la Naringine peut sensibiliser les cellules chimio/ radiorésistantes vis à vis des agents thérapeutiques conventionnels et qu'en tout cas ces cellules peuvent être détruites par l'utilisation de la Naringineet/ou de la Naringenine, ou leurs dérivés .

## Exemple 8

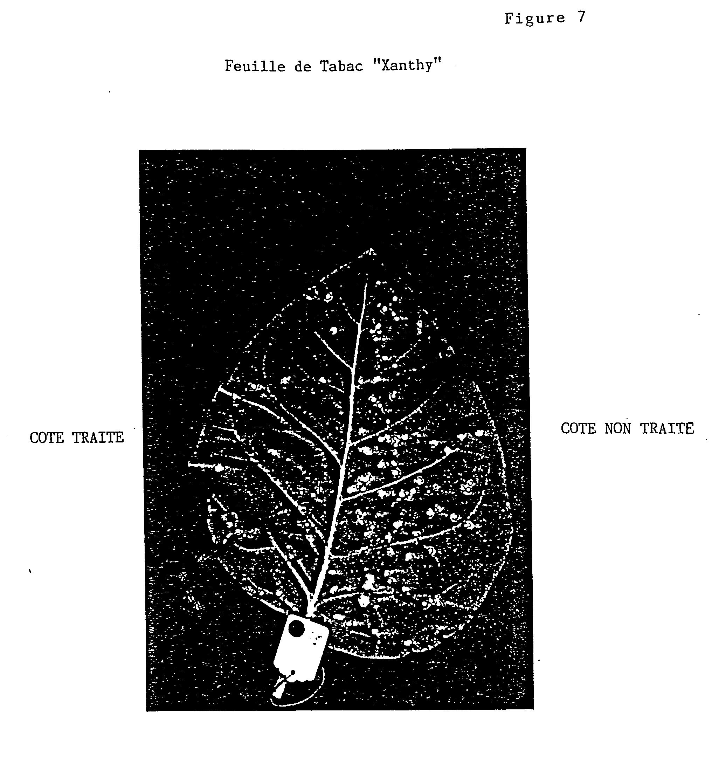

La Naringine préparée selon l' Exemple 1 ainsi que celle provenant du commerce ont été testées sur des feuilles de Tabac infectées par le virus de la mosaïque du tabac (VMT) . Dans cette expérience , une suspension virale contenant 2 µg/ml de virus en solution eau distillée , a été incubée à 30°C pendant 30 minutes avec chacun des composés cités ci-dessus (20-50 microgrammes/ millilitre) puis 20 µl sont déposés sur une moitié de feuille de tabac ("Xanthy") . La même concentration de virus , non traité cette fois , étant déposée sur l'autre moitié de la feuille . L'apparition de la maladie virale survient 2-3 jours plus tard . Le comptage des plages unitaires (PFU) , chacune correspondant à environ $10^6$ particules virales, montre que chacun des échantillons de Naringine ajouté à la suspension virale inhibe la multiplication des virus . La Figure 7 montre les résultats de ce test en utilisant la Naringine isolée selon l'Exemple 1. La Naringenine ou la Naringine commerciales donnent des résultats similaires .

Exemple 9

Toutes deux , Naringine et Naringenine inhibent in vitro l'activité de la desoxynucleotidyl transférase términale (TdT), enzyme impliquée dans l'intégration du génome viral dans la cellule hôte . Cette enzyme est présente dans le vaccin de la leucose du chat préparé par le génie génétique (vaccin leucocell Engerix [R] ) , vaccin très purifié. J'ai précédemment montré que cette enzyme catalyse la polymérisation de d-TMP et d-CMP provenant de d-TTP et d-CTP utilisés respectivement comme substrats .

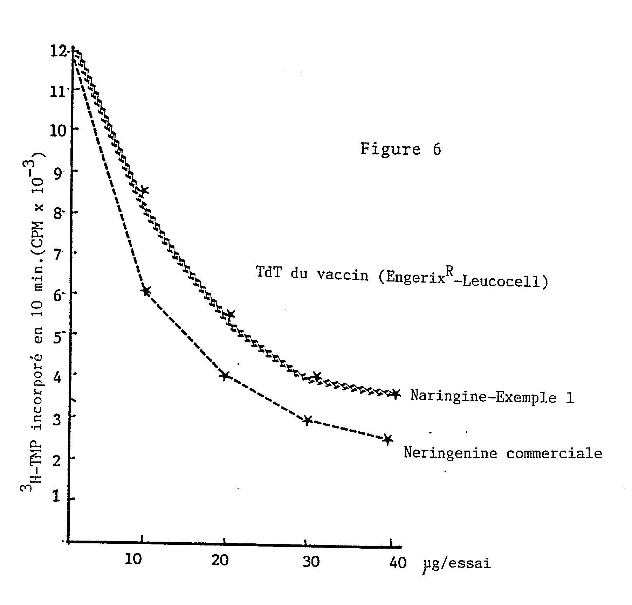

Pour l'essai d'activité TdT , le milieu d'incubation (0,10 ml de volume final) contient : 25 µmol d'une solution fraiche de tampon Tris-HCl pH 7,70 ; 2 µmol de $MgCl_2$ ; un ($^3$H)-d-NTP (désoxynucleoside-5'-triphosphate , 100.000 CPM). Des quantités croissantes de vaccin Leucocell (comme indiqué dans la figure 6) sont utilisées . Incubation 10 min à 36°C puis addition d'un égal volume d'acide trichloracétique (ATC 5%). Le matériel acido-précipité est filtré sur millipore (Whatman GF/C), lavé à l'ATC 5% puis à 'alcool 95° . Après sèchage du millipore , la radioactivité du matériel acido insoluble est mesurée dans un spectromètre à scintillation Packard. Les résultats sont exprimés en CPM (coups/minute)/échantillon.

La Figure 6 montre que la Naringine et/ou la Naringenine inhibent l'activité de la TdT qui se trouve comme contaminant dans le vaccin commercial préparé à partir d'un virus à ARN , responsable de la leucose du chat.

En revanche , l'activité de la TdT qui contamine aussi le vaccin Engerix[R] - antigène B ( Med.Sci.Res. 1987 , vol. 15 pp.529-530 ), antigène préparé à partir d'un virus à ADN , n'est pas inhibée , dans les mêmes conditions , par la Naringine ou par la Naringenine. Ainsi , toutes deux , Naringineet Naringenine interfèrent sélectivement avec la prolifération des virus à ARN mais non avec le virus à ADN. Cependant , comme l'ARN des virus à ARN est transcrit en ADN par la transcriptase inverse avant d'être intégré au génome de la cellule (rôle potentiel de la TdT), Naringine et Naringenine sont des agents utiles également dans le traitement de toute inffection à ADN également.

Exemple 10

L'effet de la Naringine sur l'activité in vitro de la transcriptase inverse fut déterminée . La transcriptase inverse commerciale provenant du virus de l'érythroblastose (virus à ARN ) fut utilisée . Le test fut pratiqué comme suit :

Le milieu réactionnel (150 µl de volume final) contenait : 25 µM de tampon Tris-HCl pH 7.7 ; 2 µM de $MgCl_2$ ; 0,6 nM de chacun des désoxyribonucleoside-5'-triphosphates : désoxyadénosine-triphosphate (dATP), désoxycytidine-triphosphate (dCTP), désoxyguanosine-triphosphate (dGTP) et 0,6 nM de $^3$H-TTP (thymidine triphosphate)(100,000 CPM) ; 0,2 à 0,5 µg de mRNA de la globine du lapin (Hg), solution fraichement préparée: 0,1 µg d'oligo dT$_{12-18}$ et 1 à 5 µg de protéine enzymatique. Après 10 min. d'incubation à 37°C la réaction est arrêtée par addition d'un égal volume d'acide trichloracétique froid (10%ATC) contenant 0,02 M de pyrophosphate de Na. L'ensemble est filtré sur filtre de verre Whatman (GF/A ou GF/C), le précipité lavé par l'ATC 5% contenant 0,02 M de pyrophosphate puis par l'alcool éthylique à 95° et enfin séché. La radioactivité est mesurée dans 5 ml de liquide à scintillation dans un compteur Beckman. La radioactivité du matériel acido-précipitable est déterminée.

Le tableau montre le taux d'inhibition produit par la présence de Naringine dans le milieu réactionnel,en présence de MRNA de globine.

Tableau

| CPM de $^3$H-TMP incorporé en 10 min | | % d'inhibition |
|---|---|---|
| milieu complet | 1676 | - |
| + Naringine (Exemple 1): | | |
| 25 µg ....... | 836 | 50 |
| 50 µg ....... | 520 | 70 |
| 150 µg ....... | 501 | 71 |

Exemple 11

Cet exemple illustre l'absence de toxicité de différentes doses de Naringine ou Naringenine injectées par voie intraveineuse à des souris BALB C.La fragilité des veines des souris ne permettait pas de pratiquer des injections plus répétées, soit pour la toxicité, soit pour le traitement des tumeurs par cette voie.

Naringine(ou Naringenine)voie i.v. (DMSO $^{10\%}$)

| 125 mg/kg ......... | aucun | effet | toxique |
|---|---|---|---|
| 250 mg/kg ......... | " | " | " |
| 500 mg/kg ......... | " | " | " |

On peut donc conclure que la Naringineou la Naringenine peuvent être administrées soit par injection i.v. soit par perfusion , à des doses de 250 - 1000 mg/kg , doses bien tolérées .

Exemple 12

La faible affinité de la Naringine pour l'ADN des cellules normales d'une part , pour les cellules en culture in vitro d'autre part ainsi que l'absence de toxicité chez la souris sont autant de faits qui suggèrent une absence de toxicité envers les cellules du sang , globules blancs et plaquettes en particulier .

Une solution de Naringine (10% de DMSO en serum physiologique) est injectée à raison de 50 mg , trois fois par semaine par voie intraveineuse , pendant deux mois consécutifs à des lapins . Aucun changement de la formule sanguine de l'animal ainsi traité n'a pu être détecté , pas plus qu'une perte de poids .

Exemple 13

Des souris ♂ BALB C porteuses du lymphome YC8 (forme ascitique) et des souris Swiss porteuses de cellules ascitiques d' Ehrlich (20-22 gr , élevage Charles River) sont réparties au hasard en lot de 10. Chaque lot recevait respectivement :

Lot -1 : Témoins, ayant reçu des cellules tumorales et une solution isotonique de NaCl (0,2 ml/souris, 2 fois/jour, voie i.p.)

Lot 2 : Souris porteuses de cellules tumorales recevant (J0-1 = Naringine exemple 1) (0,2 ml /souris 2 fois/jour). Pour les concentrations de J0-1, voir tableaux et figures ). Voie i.p.)

Lot III : Souris porteuses de cellules tumorales, recevant Naringine) (J0-1) (0,2 ml/souris 2 fois/jour ,(concentrations, voir les illustrations) et en même temps ces souris reçoivent un des produits de la chimiothérapie (concentrations, voir illustration) voie i.p.

Les cellules tumorales ascitiques sont prélévées en milieu stérile sur souris portant ces cellules depuis 15-20 jours. 0.1 ml de suspension d'ascite est mélangé avec 10 ml de solution isotonique tamponnée (pH 7 .2) : (NaCl , 7,2 g/l ; Na$_2$HPO$_4$ , 4,3 g/l et KH$_2$PO$_4$, 0,4 g/l ).Une numération des cellules est effectuée (cellule de Malassez) et la suspension cellulaire diluée afi que le nombre de cellules soit d'environ 40.000-50.000/ml. 0,1 ml de cette suspension est immédiatement injecté par voie i.p. aux souris des lots I , II et III.

Traitement : 48 h après injection des cellules tumorales, les souris du lot II reçoivent (i .p.) J0-1 réchauffé à 37° et filtré sur millipore. Traitement 5 jours consécutifs. Les souris du lot III sont traitées par le mélange de J0-1 et d'un des antimitotiques pendant 5 jours consécutifs tandis que le lot I témoin ne reçoit que du sérum physiologique pendant 5 jours. Les souris sont observées pendant les deux mois qui suivent après la cessation du traitement. Les survivants en excellente condition physique sont seuls pris en considération. Il faut toutefois préciser que les cellules ascitiques d'Ehrlich sont moins sensibles à l'action de Naringine que les cellules du lymphome YC8 .

**Revendications**

1. Utilisation d'une composition pharmaceutique renfermant un des composés sélectionnés dans le groupe constitué par la Naringine, Naringenine, leurs esters ou sels, et cela en présence d'un support pharmaceutique acceptable, pour l'obtention d'un médicament destiné à l'inhibition de la croissance des cellules cancéreuses qui comporte le contact de telles cellules avec des concentrations efficaces de ladite composition.

2. Une composition selon la revendication 1 dans laquelle la Naringine est présente.

3. Une composition selon la revendication 1 dans laquelle la Naringenine est présente.

4. Une composition pharmaceutique renfermant un mélange de Naringine, Naringenine et un support pharmaceutique acceptable.

5. Une composition selon la revendication 1, sous forme d'unité de dosage.

6. Une composition selon la revendication 2, sous forme d'unité de dosage.

7. Une composition selon la revendication 3, sous forme d'unité de dosage.

8. Utilisation d'une composition selon une des revendications 1 à 7 pour l'otention d'un médicament destiné au traitement d'un mammifère porteur de cellules cancéreuses comportant l'administration de ladite composition et ceci en quantité telle que la croissance des cellules cancéreuses sensibles à ladite composition soit inhibée plus fortement que n'est inhibée la croissance des cellules normales hématopoiétiques.

9. Utilisation selon la revendication 8 dans laquelle lesdites cellules cancéreuses sont résistantes à la chimiothérapie ou à la radiothérapie.

10. Utilisation d'une composition selon une des revendications 1 à 7 pour l'obtention d'un médicament destiné à augmenter l'efficacité du traitement chimiothérapeutique d'un mammifère porteur de cellules cancéreuses et comprenant en additif un traitement supplémentaire avec une quantité synergique de ladite composition.

11. Utilisation d'une substance selon une des revendications 1 à 7 our l'obtention d'un médicament destiné à l'inhibition des effets enzymatiques de la terminale désoxynucléotidyl transférase du virus à ARN ou de la transcriptase inverse retrovirale qui comporte l'utilisation de ladite composition dans un système biologique où se trouvent de tels enzymes.

12. Utilisation d'une substance selon une des revendications 1 à 7 pour l'obtention d'un médicament destiné à combattre l'infection virale chez l'homme et l'animal qui comporte l'administration au sujet subissant une telle infection d'une quantité active de ladite composition.

13. Utilisation d'une substance selon une des revendications 1 à 7 pour l'obtention d'un médicament destiné à combattre l'infection virale chez une plante qui comporte l'administration à la plante subissant une telle infection d'une quantité active de ladite composition.

14. Procédé d'extraction de la Naringine à partir de sources végétales naturelles contenant ces mêmes produits, ce qui comporte :

a/ préparer un extrait aqueux de la source végétale

b/ soumettre l'extrait à l'hydrolyse acide

c/ extraire le matériel aqueux par des solvents organiques afin de récupérer la Naringine en solution

d/ évaporer facultativement le solvent laissant un résidu, reprendre le résidu par un mélange eau-ethanol, ramener le pH vers la neutralité et filtrer afin de récupérer la Naringine sur le filtre

e/ ramener le pH à la neutralité si nécessaire.

15. Procédé selon la revendication 14 dont la source végétale est Hippophae Rhamnoides.

16. Procédé selon la revendication 14 dont la source végétale est le fruit citron, le pamplemousse ou l'orange.

17. Procédé selon la revendication 14 dont la source végétale est les racines d'endives.

18. Utilisation d'une composition selon une des revendications 1 à 7 pour l'obtention d'un médicament destiné à la contraction sélective des chaines d'ADN des cellules cancéreuses, alors que la contraction des chaines d'ADN des cellules normales saines ne se fait pas, ce qui comporte de soumettre un système biologique contenant de telles chaines à l'action d'une concentration efficace de ladite composition.

TABLEAU 1  Survie des souris ♂(BALB C) porteuses du lymphome YC8, non traitées et traitées par la Naringine commerciale (voie i.p.) et Naringine exemple 1 .

| Survie(jours) | non traitées | Naringine commerciale 2 mg x 2/j | Survie % | Naringine Exemple 1 2 mg x 2/j | Survie % |
|---|---|---|---|---|---|
| 0 | 10 | 10 | 100 | 10 | 100 |
| 10 | 10 | 10 | 100 | 10 | 100 |
| 15 | 7 | 8 | 80 | 10 | 100 |
| 20 | 2 | 5 | 50 | 8 | 80 |
| 25 | 0 | 5 | 50 | 7 | 70 |
| 30 | 0 | 5 | 50 | 7 | 70 |

EP 0 352 147 A2

TABLEAU 2   Survie des souris ♂ (BALB C) porteuses du lymphome YC8 non traitées et traitées par la Naringine( voie i.m.)

| Survie( jours) | non traitées | Naringine (Exemple 1) 3 mg x 2/j durant 15 jours | Survie (%) |
|---|---|---|---|
| 0 | 20 | 20 | 100 |
| 10 | 20 | 20 | 100 |
| 20 | 12 | 20 | 100 |
| 25 | 0 | 17 | 85 |
| 60 | 0 | 17 | 85 |

EP 0 352 147 A2

TABLEAU 3  Survie des souris ♂ Swiss porteuses de cellules ascitiques d'Ehrlich , non traitées et traitées par la Naringine (Exemple 1) (voie i.p.)

| Survie (jours) | non traitées | NARINGINE exemple 1 | | | |
|---|---|---|---|---|---|
| | | 1,5 mg x 2/j | Survie % | 3.0 mg x 2/j | Survie % |
| 0 | 10 | 10 | 100 | 10 | 100 |
| 10 | 10 | 10 | 100 | 10 | 100 |
| 15 | 8 | 4 | 40 | 10 | 100 |
| 20 | 4 | 4 | 40 | 7 | 70 |
| 25 | 1 | 4 | 40 | 5 | 50 |
| 30 | 0 | 4 | 40 | 5 | 50 |

EP 0 352 147 A2

TABLEAU 4  Survie des souris ♂ BALB C porteuses de cellules ascitiques du lymphome YC8 , non traitées et traitées par la Naringenine commerciale (voie i.p.)

| Survie jours | non traitées | NARINGENINE commerciale | | | |
|---|---|---|---|---|---|
| | | 0,5 mg x 2/j | survie % | 1 mg x 2/j | survie % |
| 0 | 10 | 10 | 100 | 10 | 100 |
| 10 | 10 | 10 | 100 | 10 | 100 |
| 15 | 10 | 10 | 100 | 10 | 80 |
| 20 | 6 | 7 | 70 | 8 | 50 |
| 25 | 2 | 3 | 30 | 5 | 50 |
| 30 | 0 | 3 | 30 | 5 | 50 |

EP 0 352 147 A2

Figure 1

FIGURE 2

Figure 3

Moelle osseuse ⎫
Rate ⎬ ADN normal humain
DNA de cerveau de singe ⎭

Sein ⎫ ADN humains
Colon ⎬ cancéreux
Foie ⎭

$^3$H-ADN SYNTHETISE (%)

NARINGINE-exemple 1 ( µg/essai).

EP 0 352 147 A2

Figure 4

$^3$H-ADN SYNTHETISE (%)

Contrôle

NARINGINE-Exemple 1 (50 µg/essai)

NARINGINE-Exemple 1 (50 µg/essai)

TEMPS (min.)

EP 0 352 147 A2

Figure 5

HYPOCHROMICITE DE DIFFERENTS ADN

NARINGINE-EXEMPLE-1

NARINGENINE (ECHANTILLON COMMERCIAL)

Figure 6

Figure 7

Feuille de Tabac "Xanthy"

COTE TRAITE

COTE NON TRAITE

NARINGINE

naringenine

Figure 8

EP 0 352 147 A2

Figure 9

EP 0 352 147 A2

SOURIS PORTEUSES DES CELLULES ASCITIQUES D'EHRLICH :
5-FU :10 µg x 2 ; JO-1: 1,5 mg x 2 (I.P., 5 jours)

SOURIS PORTEUSES DES CELLULES ASCITIQUES D'EHRLICH:
ARA-C: 10 µg x 2 ; JO-1 : 1,5 mg x. 2 (I.P.5 jours)

Figure 10

Souris porteuses de lymphome YC8. Deticene :
100 µg x 2 ; JO-1 : 1,5 mg x 2 (I.P.,5 jours)

Souris porteuses de lymphome YC8. ARA-C :
10 µg  x 2 ; JO-1 : 1,5 mg x2 (I.P.,5 jours)

Souris porteuses de lymphome YC8.Endoxan :
10 µg x 2 ; JO-1 : 1,5 mg x 2 (I.P.,5 jours)

Souris porteuses de lymphome YC8. HU:
10 µg x 2 ; JO-1 : 1,5 mg x 2 (I.P.,5 jours)

EP 0 352 147 A2